# FASCICULE DE BREVET EUROPEEN

(11) **EP 3 194 350 B1**
(45) Date de publication et mention de la délivrance du brevet: **07.11.2018**
(21) Numéro de dépôt: 15771060.9
(22) Date de dépôt: 17.09.2015
(51) Int. Cl.: C07C 1/20

(54) **PROCEDE DE PRODUCTION DE BUTADIENE A PARTIR D'ETHANOL EN UNE ETAPE REACTIONNELLE A FAIBLE CONSOMMATION EN EAU ET EN ENERGIE**
VERFAHREN ZUR HERSTELLUNG VON BUTADIEN AUS ETHANOL IN EINEM REAKTIONSSCHRITT MIT GERINGEM WASSER- UND GERINGEM ENERGIEVERBRAUCH
METHOD FOR THE PRODUCTION OF BUTADIENE FROM ETHANOL IN ONE LOW-WATER- AND LOW-ENERGY-CONSUMPTION REACTION STEP

(30) Priorité: 19.09.2014 FR 1458860
(43) Date de publication de la demande: 26.07.2017
(73) Titulaire: IFP Energies nouvelles, 92500 Rueil-Malmaison (FR); Compagnie Générale des Etablissements Michelin, 63000 Clermont-Ferrand (FR)
(72) Inventeur: DASTILLUNG, Rejane, 69005 Lyon (FR); FISCHER, Beatrice, 69005 Lyon (FR); JACQUIN, Marc, 69002 Lyon (FR); HUYGHE, Raphael, 69700 Saint Andeol Le Chateau (FR)
(74) Mandataire: IFP Energies nouvelles
(86) Numéro de dépôt international: PCT/EP2015/071362
(87) Numéro de publication internationale: WO 2016/042096

(56) Documents cités:
- US-A- 2 403 743

## Description

### DOMAINE TECHNIQUE DE L'INVENTION

L'invention concerne un procédé de production de butadiène à partir d'éthanol ou d'un mélange éthanol/acétaldéhyde opérant en une étape réactionnelle.

### ART ANTÉRIEUR

Les procédés de production de butadiène à partir d'éthanol ont été développés, en particulier, par les Russes sur la base des travaux de Lebedev dans les années 20 (procédé en 1 étape réactionnelle), et par les Américains durant la seconde guerre mondiale à partir des travaux d'Ostromilenski (procédé en 2 étapes réactionnelles : déshydrogénation d'éthanol en acétaldéhyde, puis production du butadiène à partir d'un mélange éthanol/acétaldéhyde).

Le procédé, dans sa version Lebedev, a une conversion par passe largement inférieure à 50%, ce qui implique des recyclages importants et compliqués pour ajuster exactement le ratio éthanol/acétaldéhyde à l'entrée de la section réactionnelle.

Un autre problème du procédé est la production d'une grande variété d'impuretés de toutes sortes: des hydrocarbures saturés, insaturés, et aromatiques, mais aussi des produits oxygénés tels que des alcools, des phénols, des aldéhydes, des cétones, des acides, des esters, des éthers, des acétals.

Certains de ces sous-produits, tant gazeux que liquides dans les conditions normales de température et de pression, sont générés en quantité significative. Parmi les sous-produits gazeux, on peut citer l'hydrogène, le monoxyde de carbone, le dioxyde de carbone, les alcanes et oléfines en C₁-C₄, le méthyle éthyle éther. Parmi les sous-produits liquides, on peut citer les pentènes, les pentadiènes, le diéthyle éther, l'éthyle vinyl éther, les hexènes, les hexadiènes, le butanal, le crotonaldéhyde, l'acétate d'éthyle, le diéthyle acétal, le butanol, l'hexanol, l'acide acétique. Ces sous-produits gazeux et liquides sont problématiques pour l'obtention d'un butadiène aux spécifications, mais aussi car leur recyclage vers les étapes réactionnelles avec l'éthanol et l'acétaldéhyde induit une diminution du rendement global de l'unité. Leur extraction complique grandement le procédé de séparation.

D'autres sous-produits sont générés en quantité infimes. Dans la suite du document, on parlera d'"huiles brunes" pour designer l'ensemble de milliers de composés hydrocarbures et oxygénés produits dans les sections réactionnelles dont les températures d'ébullition sont comprises entre celle de l'éthanol et allant jusqu'à 600°C. Ces huiles brunes ont la particularité d'être solubles dans l'éthanol, mais insolubles dans l'eau. Elles peuvent, partout où elles ne sont pas diluées par un fort excès d'éthanol, encrasser et boucher les équipements. Par ailleurs, ces huiles brunes sont problématiques au sein de la colonne à distiller qui sépare l'eau produite par la réaction et l'éthanol non converti. En effet, ces huiles brunes sont solubles dans l'effluent eau-éthanol alimentant ladite colonne à distiller, et insolubles dans le résidu essentiellement constitué d'eau. Une séparation de phase se produit donc au sein de cette colonne à distiller, diminuant considérablement l'efficacité de la séparation. Les huiles brunes sont difficiles à éliminer au sein du procédé du fait qu'elles sont constituées de milliers de composés ayant des propriétés physico-chimique très différentes. Une fraction de ces huiles brunes s'accumule donc au sein du procédé, entrainant une baisse de son efficacité au bout de quelques jours, au mieux semaines, d'opération. Il est donc nécessaire de purger périodiquement certains flux, entrainant une perte en éthanol et en acétaldéhyde dégradant ainsi le rendement global du procédé pour un coût qui serait aujourd'hui rédhibitoire.

Du fait des nombreuses impuretés produites par le procédé, la purification du butadiène est complexe. Elle fait appel à une combinaison de nombreuses opérations unitaires, telles que des lavages, des distillations simples et extractives. L'art antérieur enseigne l'utilisation de distillations extractives mettent en oeuvre un solvant bis(2-chloroéthyle)éther (Chlorex), aujourd'hui proscrit du fait de sa forte toxicité. Il est important de noter que les spécifications du butadiène sont aujourd'hui extrêmement sévères, de par la sensibilité des catalyseurs de polymérisation du butadiène. La reproduction de l'enchainement des opérations unitaires selon l'art antérieur ne permettrait donc pas d'atteindre les spécifications actuelles. Par exemple, la spécification en acétaldéhyde (réactif intermédiaire permettant de produire le butadiène) dans le butadiène est passée de 1000 ppm à moins de 10 ppm aujourd'hui. L'ouvrage « Synthetic rubber from Alcohol», (A. Talalay, M. Magat, 1945) donne une vue générale du procédé développé.

US 2,403,743 décrit un procédé cyclique de production de butadiène à partir d'un mélange d'éthanol et d'acétaldéhyde, ainsi que les moyens de récupération et de recyclage de l'acétaldéhyde dans le procédé.

### OBJET ET INTÉRÊT DE L'INVENTION

L'invention a pour objet un procédé de production de butadiène à partir d'une charge éthanol comprenant au moins 80% poids d'éthanol comprenant au moins :
A) une étape de conversion de l'éthanol en butadiène comprenant au moins une section réactionnelle, alimentée au moins par l'effluent éthanol et une fraction de l'effluent acétaldéhyde issus de l'étape E), opérée à une pression comprise entre 0,1 et 1,0 MPa et à une température comprise entre 300 et 400°C en présence d'un catalyseur, et une section de séparation permettant de séparer l'effluent de ladite section réactionnelle en au moins un effluent gazeux et un effluent liquide, la fraction de l'effluent acétaldéhyde issu de l'étape E) n'alimentant pas ladite section réactionnelle constituant le flux de purge ;
B) une étape d'extraction du butadiène comprenant au moins une section de compression comprimant ledit effluent gazeux issu de l'étape A) à une pression comprise entre 0,1 et 1,0 MPa, une section de lavage gaz-liquide alimentée par un flux éthanol constitué de ladite charge éthanol du procédé et/ou une fraction de l'effluent éthanol issu de l'étape E), et par ledit effluent gazeux comprimé, dans laquelle l'alimentation gaz est réalisée à une température comprise entre 10 et 60°C, et l'alimentation liquide est réalisée à une température comprise entre 20°C et - 30°C, et produisant au moins un flux éthanol enrichi en butadiène et un effluent sous-produits gazeux, et une section de distillation alimentée par ledit flux éthanol enrichi en butadiène et produisant un effluent butadiène brut et un résidu éthanol/acétaldéhyde/eau, ladite section de distillation étant opérée entre 0,1 et 1 MPa ;
C) une étape de lavage à l'eau des sous-produits gazeux, alimentée par l'effluent sous-produits gazeux issu de l'étape B), ainsi que par une fraction de l'effluent riche en eau issu de ladite étape E) et produisant au moins un effluent eau alcoolisée ;
D) une étape d'élimination des impuretés et des huiles brunes, alimentée au moins par l'effluent éthanol/acétaldéhyde/eau issu de l'étape B), et par une fraction de l'effluent eau issu de l'étape E), et produisant au moins un raffinat eau / éthanol / acétaldéhyde, un effluent huiles brunes légères et un effluent huiles brunes lourdes ;
E) une étape traitement des effluents alimentée au moins par le raffinat eau / éthanol / acétaldéhyde issu de l'étape D), et produisant au moins un effluent éthanol, un effluent acétaldéhyde et un effluent eau ;
F) une étape de première purification du butadiène comprenant au moins une section de lavage gaz-liquide alimentée en fond par l'effluent butadiène brut issu de B) et en tête par un flux d'eau pouvant être un flux d'eau d'origine externe audit procédé de production de butadiène et/ou une fraction de l'effluent eau issu de l'étape E), ladite section de lavage produisant en tête un effluent butadiène pré-purifié et en fond un effluent eau usée ;
G) une étape ultérieure de purification du butadiène, alimentée au moins par ledit effluent butadiène pré-purifié issu de ladite étape F), et produisant au moins un effluent butadiène purifié.

La demanderesse a identifié un agencement d'opération unitaires, qui permettent de palier à de nombreux inconvénients de l'art antérieur. En particulier, l'agencement des opérations unitaires selon l'invention permet d'éliminer les impuretés gazeuses, les impuretés liquides et les huiles brunes tout en minimisant la perte en éthanol et acétaldéhyde, améliorant ainsi le rendement global de l'unité tout en réduisant le flux global d'eau nécessaire aux étapes de séparation et en obtenant un butadiène très pur. La réduction importante du flux global d'eau permet de réduire la consommation énergétique du procédé et la taille des équipements de séparation.

### DESCRIPTION DÉTAILLÉE DE L'INVENTION

### Charge

La charge éthanol utilisée dans le procédé selon l'invention peut provenir de toute origine, fossile, végétale ou animale, et en particulier des procédés de production d'éthanol à partir de ressources végétale. Ladite charge comprend au moins 80% poids d'éthanol, préférentiellement au moins 90% poids, et de manière préférée au moins 93 % poids. De manière très préférée, ladite charge éthanol répond aux spécifications d'éthanol carburant EN 15376.

### Étape A) de conversion de l'éthanol en Butadiène

Conformément à l'invention, une étape A) de conversion de l'éthanol en butadiène comprend au moins une section réactionnelle et une section de séparation permettant de séparer l'effluent de ladite section réactionnelle en au moins un effluent gazeux et un effluent liquide. Ladite section réactionnelle de ladite étape A) est alimentée par l'effluent éthanol et l'effluent acétaldéhyde issus de l'étape E) de traitement des effluents, et optionnellement par une fraction de ladite charge éthanol. Ladite étape A) peut également être alimentée par un flux externe d'acétaldéhyde.

Le ratio éthanol sur acétaldéhyde à l'entrée de la section réactionnelle est ajusté en contrôlant la fraction de l'effluent acétaldéhyde issu de ladite étape E) alimentant l'étape A). Le contrôle du ratio éthanol sur acétaldéhyde dans ladite section réactionnelle est donc aisé avec le procédé selon l'invention. Ce ratio est ajusté à la valeur désirée par l'Homme du métier, en fonction du catalyseur mis en oeuvre.

La fraction de l'effluent acétaldéhyde issu de ladite étape E) qui n'est pas envoyé dans la section réactionnelle constitue un flux dénommé purge. L'acétaldéhyde et l'éthyle acétate contenus dans la purge pourront être transformés dans une étape d'hydrogénation dédiée pour produire de l'éthanol qui sera alors envoyé vers la dite section réactionnelle de ladite étape A). Elle peut également être traitée dans une étape réactionnelle dédiée contenant un catalyseur de type silice avec un oxyde de tantale, de zirconium pour produire du butadiène, opérée à une température comprise entre 300 et 400°C et à une pression comprise entre 0,1 et 1,0 MPa. De manière préférée, le débit de purge est nul et l'intégralité de l'effluent éthanol/acétaldéhyde issu de ladite étape E) est envoyé dans la section réactionnelle.

Ladite section réactionnelle permet de convertir une partie du mélange éthanol/acétaldéhyde en au moins du butadiène. Elle est opérée en présence d'un catalyseur bien connu de l'Homme du métier, par exemple un catalyseur de type silice avec un oxyde de magnésium, à une température comprise entre 300 et 400°C, de manière préférée entre 320 et 370°C et à une pression comprise entre 0,1 et 1,0 MPa, de préférence entre 0,1 et 0,5 MPa, de manière préférée entre 0,1 et 0,3 MPa. La sélectivité de la transformation du mélange éthanol/acétaldéhyde est préférentiellement supérieure à 20%, de manière préférée supérieure à 25%, de manière très préférée supérieure à 50%. Par sélectivité, on entend le rapport molaire du débit de butadiène dans l'effluent de ladite section réactionnelle sur le débit d'éthanol et d'acétaldéhyde consommé dans ladite section réactionnelle. La conversion de la transformation du mélange éthanol/acétaldéhyde est préférentiellement supérieure à 40%, de manière préférée supérieure à 50%, de manière préférée supérieure à 60%. Par conversion, on entend le rapport molaire du débit d'éthanol et d'acétaldéhyde dans l'effluent de ladite section réactionnelle sur le débit d'éthanol et d'acétaldéhyde dans l'alimentation de ladite section réactionnelle.

Pour maximiser la conversion de l'éthanol et acétaldéhyde et la sélectivité en butadiène, les compositions en entrée de la section réactionnelle sont ajustées en fonction du catalyseur sélectionné.

L'effluent de ladite section réactionnelle comprend encore de l'éthanol, ainsi que de nombreuses impuretés produites avec le butadiène, parmi lesquelles de l'éthylène, du propylène, du diéthyl ether (DEE), de l'acétate d'éthyle, du butanol, de l'hexanol, des butènes, des pentènes, des pentadiènes, des hexènes, et des hexadiènes. Il alimente ladite section de séparation de ladite étape A).

Ladite section de séparation a pour objectif d'extraire les constituants incondensables de l'effluent liquide. Elle produit un effluent gazeux, qui contient principalement du butadiène, de l'hydrogène, de l'éthanol, de l'acétaldéhyde et des hydrocarbures légers, et un effluent liquide, qui contient principalement de l'eau, de l'éthanol, de l'acétaldéhyde et des hydrocarbures lourds.

Ladite section de séparation met en oeuvre des moyens de séparation gaz-liquide connus de l'Homme du métier. De manière préférée, on utilisera un séparateur gaz-liquide opéré à une pression comprise entre 0,1 et 0,3 MPa, et une température comprise entre 10 et 60°C.

### Étape B) d'extraction du butadiène

Conformément à l'invention, une étape B) d'extraction du butadiène comprenant au moins une section de compression, une section de lavage gaz-liquide, et une section de distillation est alimentée par ledit effluent gazeux issu de l'étape A), éventuellement par ledit effluent liquide issu de l'étape A) et par un flux éthanol constitué de ladite charge éthanol du procédé et/ou d'une fraction de l'effluent éthanol issu de l'étape E). Ladite étape B) produit au moins un effluent sous-produits gazeux, un effluent butadiène brut, et un effluent éthanol/acétaldéhyde/eau.

Ledit flux éthanol alimentant l'étape B) comprend au moins 80% poids d'éthanol, préférentiellement au moins 90% poids, et de manière préférée au moins 93 % poids. Ledit flux éthanol alimentant l'étape B peut contenir du méthanol, de l'eau, de l'acétate d'éthyle, du butanol et de l'hexanol. De préférence, ledit flux éthanol alimentant l'étape B comprend moins de 10% poids d'acétaldéhyde, de préférence moins de 5% poids, et de manière préférée moins de 1% poids. De préférence, ledit flux éthanol alimentant l'étape B comprend moins de 20% poids d'eau, de préférence moins de 5% poids, de manière préférée moins de 1% poids.

Dans un autre arrangement préféré, ledit flux éthanol est constitué d'une fraction de l'effluent éthanol issu de l'étape E) de traitement des effluents.

L'utilisation d'un flux éthanol contenant peu ou pas d'acétaldéhyde minimise l'entraînement d'acétaldéhyde dans ledit effluent sous-produits gazeux soutiré en tête de ladite section de lavage gaz-liquide réduisant les pertes en rendement global du procédé, ainsi que le débit d'eau de lavage nécessaire dans l'étape C) de lavage à l'eau des sous-produits gazeux.

L'effluent gazeux issu de l'étape A) est comprimé dans ladite section de compression à une pression comprise entre 0,1 et 1,0 MPa, préférentiellement entre 0,1 et 0,7 MPa, et de manière préférée entre 0,2 et 0,5 MPa. L'effet de cette compression est d'une part de diminuer le débit volumique de gaz, et d'autre part d'améliorer l'efficacité du lavage en aval. L'effluent gazeux comprimé est ensuite refroidi à une température comprise entre 10 et 60°C, préférentiellement entre 30°C et 40°C.

Ladite section de lavage gaz-liquide de l'étape B) comprend une colonne de lavage alimentée en tête par ledit flux éthanol alimentant l'étape B), en fond par ledit effluent gazeux comprimé et refroidi et produit en tête l'effluent sous-produits gazeux et en fond un flux éthanol enrichi en butadiène qui alimente ladite section de distillation de l'étape B).

Ledit flux éthanol alimentant l'étape B) est refroidi avant d'être alimenté en tête de ladite colonne de lavage gaz-liquide de la section de lavage à une température comprise entre 20 et - 30°C, préférentiellement entre 5°C et - 15°C. L'intérêt de refroidir ledit flux éthanol est d'améliorer la performance de l'opération de lavage en minimisant l'entraînement d'éthanol et d'acétaldéhyde dans ledit effluent sous-produits gazeux. Ainsi, la totalité du butadiène présent dans l'effluent gazeux issu de l'étape A comprimé et refroidi est abattue, et l'effluent sous-produits gazeux soutiré en tête de ladite section de lavage gaz-liquide est exempt de butadiène.

La minimisation de l'entraînement d'acétaldéhyde dans ledit effluent sous-produits gazeux permet, incidemment, de diminuer de manière importante le débit d'eau requis dans l'étape C) de lavage à l'eau des sous-produits gazeux, dont l'objectif est de récupérer l'éthanol et les traces éventuelles d'acétaldéhyde entraînés dans l'effluent sous-produits gazeux soutiré en tête de la section de lavage à l'éthanol de l'étape B).

Dans un arrangement particulier, le flux éthanol enrichi en butadiène soutiré en fond de ladite section de lavage gaz-liquide de l'étape B) est mélangé à l'effluent liquide issu de l'étape A) de manière à former l'alimentation de ladite section de distillation de l'étape B).

Dans un autre arrangement particulier, le flux éthanol enrichi en butadiène soutiré en fond de ladite section de lavage gaz-liquide de l'étape B) constitue l'alimentation de ladite section de distillation de l'étape B), l'effluent liquide issu de l'étape A) alimentant alors directement l'étape D) d'élimination des impuretés liquides et des huiles brunes. Ce dernier mode de réalisation est rendu possible par le procédé selon l'invention car l'effluent liquide issu de l'étape A) contient peu de butadiène.

Ladite alimentation est traitée dans ladite section de distillation de l'étape B) de manière à séparer en tête un effluent vapeur comprenant la majorité du butadiène, appelé effluent butadiène brut, et en fond un résidu éthanol/acétaldéhyde/eau. Par la majorité, on entend plus de 80% du butadiène compris dans l'alimentation de ladite section de distillation, préférentiellement plus de 90%, de manière préférée plus de 95%, de manière encore plus préférée plus de 98%, de manière très préférée plus de 99% et de manière très avantageuse la totalité du butadiène compris dans ladite alimentation. Ce résidu éthanol/acétaldéhyde/eau comprend de l'éthanol et de l'acétaldéhyde n'ayant pas réagi, et comprend également de l'eau produite et des sous-produits formés dans l'étape A), comme par exemple le diéthyléther et l'acétate d'éthyle et les huiles brunes. Ledit résidu éthanol/acétaldéhyde/eau alimente ensuite les étapes D) et E) de traitement des effluents. Ladite section de distillation est opérée entre 0,1 et 1 MPa et de manière préférée entre 0,2 et 0,5 MPa.

L'agencement des recyclages et l'utilisation des flux externes (charge éthanol, eau) selon l'invention, et en particulier d'un flux éthanol refroidi, permet de minimiser le débit de l'effluent eau usée, et donc le débit à traiter par lesdites étapes C) et E). Le procédé selon l'invention permet donc de minimiser le débit des effluents à traiter dans l'étape de traitement des effluents.

### Étape C) de lavage à l'eau des sous-produits gazeux

Conformément à l'invention, une étape C) de lavage à l'eau des sous-produits gazeux est alimentée par l'effluent sous-produits gazeux issu de l'étape B), ainsi que par une fraction de l'effluent riche en eau issu de ladite étape E) et produit au moins un effluent eau alcoolisée.

L'objectif de ladite étape C) est de récupérer la petite fraction d'éthanol entraînée dans ledit effluent sous-produits gazeux issu de l'étape B) afin d'améliorer le rendement global du procédé.

La quantité d'eau issue de ladite étape E) nécessaire dans ladite étape C) selon l'invention est très faible, contrairement à celle nécessaire dans l'art antérieur, car l'effluent vapeur issu de l'étape A) a été lavé dans l'étape B) avec un flux éthanol contenant peu ou pas d'acétaldéhyde. Il ne reste donc dans ce flux qu'une petite fraction d'éthanol, facilement récupérée avec une faible quantité d'eau en comparaison avec la quantité d'eau qui aurait été nécessaire s'il y avait de l'acétaldéhyde dans l'effluent sous-produits gazeux issu de l'étape B).

L'eau, chargée en éthanol après le lavage, est soutirée de ladite étape C) et constitue l'effluent eau alcoolisée. Elle alimente l'étape E), directement dans la section de distillation eau-éthanol sans alourdir la section de distillation de l'acétaldéhyde. Dans un autre mode de l'invention, elle alimente l'étape D) d'élimination des impuretés et des huiles brunes.

Le procédé selon l'invention permet donc de minimiser le débit des effluents à traiter dans l'étape de traitement des effluents. Il permet par ailleurs de réduire au maximum les pertes en butadiène, en permettant de récupérer de préférence plus de 98%, préférentiellement plus de 99 % du butadiène produit à l'issue des étapes réactionnelles dans ledit effluent butadiène purifié.

### Étape D) d'élimination des impuretés liquides et des huiles brunes

Conformément à l'invention, une étape D) d'élimination des impuretés et des huiles brunes est alimentée au moins par l'effluent éthanol/acétaldéhyde/eau issu de l'étape B) et par une fraction de l'effluent eau issu de l'étape E) et produit au moins un raffinat éthanol / acétaldéhyde / eau, un effluent huiles brunes légères et un effluent huiles brunes lourdes. Ladite étape D) peut également être alimentée par l'effluent liquide issu de l'étape A).

Ladite étape D) comprend préférentiellement au moins une section de lavage/contre-lavage, une section de distillation des huiles brunes légères, et une section de distillation des huiles brunes lourdes.

Ladite section de lavage/contre-lavage, préférentielle, est alimentée en un point intermédiaire par ledit effluent éthanol/acétaldéhyde/eau issu de l'étape B), avantageusement en mélange avec l'effluent eau usée issu de l'étape E), l'effluent eau alcoolisée issu de l'étape C) et/ou en mélange avec une fraction de l'effluent eau usée issu de l'étape F). L'effluent liquide issu de l'étape A) peut également optionnellement avantageusement alimenter ladite section de lavage/contre-lavage en un point intermédiaire. Ces effluents étant plus riches en eau que l'effluent éthanol/acétaldéhyde/eau issu de l'étape B), leur introduction en mélange permet de limiter les pertes en hydrocarbure dans le raffinat.

Ladite section de lavage/contre-lavage, préférentielle, est alimentée en fond par un effluent hydrocarbures et en tête par une fraction de l'effluent eau issue de l'étape E), qui ne comprend pas d'éthanol et d'acétaldéhyde. L'effluent hydrocarbures et la fraction de l'effluent eau issu de l'étape E) sont alimentés à une température comprise entre 10 et 70°C, préférentiellement entre 45 et 55°C. Ladite section de lavage/contre-lavage produit en tête un extrait hydrocarbures de lavages chargé d'une fraction des impuretés et des huiles brunes, et en fond ledit raffinat éthanol / acétaldéhyde / eau.

Ladite section de lavage/contre-lavage, préférentielle, est opérée à une pression de préférence comprise entre 0,1 et 0,5 MPa, préférentiellement entre 0,2 et 0,4 MPa. De préférence l'ajout d'eau pour réaliser le contre-lavage est tel que la teneur en eau dans le raffinat éthanol/acétaldéhyde/eau est supérieure à 30% poids, de manière préférée supérieur à 40% poids.

Dans un mode de réalisation, le contact entre les deux phases liquides dans ladite section de lavage/contre-lavage est réalisé au sein d'un extracteur liquide-liquide. Différents modes de contact peuvent être envisagés. On peut citer de manière non limitative, une colonne garnie, une colonne puisée, ou bien une colonne compartimentée agitée. Dans un autre mode de réalisation, le contact entre les deux phases liquides dans ladite section de lavage/contre-lavage est réalisé au sein d'un contacteur membranaire, ou une cascade de contacteurs membranaires. Ce mode de contact est particulièrement bien adapté au système mis en oeuvre. En effet, les mélanges eau-éthanol-hydrocarbure sont connus pour former des émulsions stables, qui peuvent être problématique dans un extracteur liquide-liquide. Le contacteur membranaire permet de générer une aire de contact importante, favorisant le transfert des impuretés et des huiles vers la phase hydrocarbure, sans générer d'émulsion.

Ledit extrait hydrocarbures de lavages alimente préférentiellement ladite section de distillation des huiles brunes légères, laquelle produit en tant que distillat ledit effluent huiles brunes légères, et un résidu hydrocarbures comprenant la fraction lourde des huiles brunes.

Ledit effluent huiles brunes légères est composé d'impuretés produites par l'étape réactionnelle A), principalement du diéthyléther, de l'acétate d'éthyle, du pentène, de l'isoprène, du butanal, du vinyl ethyl ether, et de la fraction légère des huiles brunes. Cet effluent peut être brulé pour fournir une partie de la chaleur nécessaire au circuit d'huile chaude ou aux chaudières à vapeur du procédé, ou distillé pour récupérer un effluent diéthyléther et/ou un effluent acétate d'éthyle, qui pourra être soit valorisée, soit recyclée dans la section réactionnelle de l'étape A) pour être retransformé.

Ledit résidu hydrocarbures contient essentiellement les hydrocarbures servant au lavage, mais également la fraction la plus lourde des huiles brunes. De préférence, pour éviter l'accumulation de la fraction lourde des huiles brunes par le recyclage de l'effluent hydrocarbures vers la section de lavage/contre-lavage, une fraction dudit résidu hydrocarbures est traitée dans ladite section de distillation des huiles brunes lourdes, consistant en une colonne à distiller, laquelle produit un distillat hydrocarbures composé pour l'essentiel d'hydrocarbures avec encore quelques traces d'huiles brunes et, en tant que résidu, ledit effluent huiles brunes lourdes comprenant de préférence plus de 80%, préférentiellement plus de 85% d'hydrocarbures ainsi que les huiles brunes les plus lourdes. De préférence, la fraction dudit résidu hydrocarbures envoyée vers ladite section de distillation des huiles brunes lourdes est comprise entre 5 et 30% du débit total dudit résidu hydrocarbures, et préférentiellement entre 10 et 25%. Le distillat hydrocarbures est préférentiellement mélangé à la fraction du résidu hydrocarbures qui n'a pas été traité dans ladite section de distillation des huiles lourdes afin de former l'effluent hydrocarbures alimentant ladite section de lavage/contre-lavage.

Ledit effluent huiles brunes lourdes, qui représente typiquement entre 0,1 et 20% de la charge de ladite section de distillation des huiles brune lourdes, préférentiellement entre 0,3 et 10%, peut être brûlé pour fournir une partie de la chaleur nécessaire au circuit d'huile chaude ou aux chaudières à vapeur du procédé. Un appoint d'hydrocarbures équivalent aux pertes en fond de ladite section de distillation des huiles lourdes peut être nécessaire pour maintenir le débit de lavage constant. Cette colonne est préférentiellement réglée de manière à maintenir constante la concentration en huiles brunes dans la boucle de recyclage des hydrocarbures (boucle effluent hydrocarbures / extrait hydrocarbures de lavage).

Les effluents huiles brunes légères et lourdes sont de préférence éliminés du procédé.

L'effluent éthanol/acétaldéhyde/eau issu de l'étape B) comprend principalement de l'éthanol, de l'acétaldéhyde, de l'eau mais aussi des impuretés telles que le diethyléther, l'acétate d'éthyle et les huiles brunes telles que définies précédemment. Ces impuretés peuvent s'accumuler si elles sont renvoyées vers l'étape réactionnelle A) au sein de la coupe de distillation riche en acétaldéhyde et/ou de la coupe de distillation riche en l'éthanol et qu'elles ne sont que partiellement converties dans la section réactionnelle de l'étape A). L'étape D) permet de récupérer une partie de ces impuretés avant l'étape E) de traitements des effluents, ce qui permet d'éviter la démixtion des huiles brunes au sein des colonnes à distiller, de simplifier le schéma de distillation, et d'obtenir à l'issue de l'étape E) un effluent éthanol, un effluent acétaldéhyde/éthanol et un effluent eau de plus grande pureté par rapport à l'art antérieur.

Dans la section de lavage/contre-lavage, préférentielle, le lavage de l'effluent éthanol/acétaldéhyde/eau issu de l'étape B) avec un effluent hydrocarbures entraîne les huiles brunes et certaines impuretés, tandis que le contre-lavage du flux d'hydrocarbures permet de limiter toute perte en acétaldéhyde et en éthanol.

De manière surprenante, la demanderesse a découvert qu'il était possible d'obtenir une séparation de phase liquide-liquide en ajoutant certains hydrocarbures au résidu éthanol/acétaldéhyde issu de l'étape B). Ce résultat est surprenant car le résidu éthanol/acétaldéhyde issu de l'étape est très riche en éthanol et acétaldéhyde qui sont miscibles en toute proportion avec les hydrocarbures. Par une sélection adéquate de l'hydrocarbure, la demanderesse a découvert qu'il était possible d'obtenir une séparation de phase liquide-liquide, et donc de réaliser une extraction liquide-liquide pour éliminer une partie des impuretés contenues dans l'effluent éthanol/acétaldéhyde/eau issu de l'étape B). Ledit effluent hydrocarbures peut contenir des hydrocarbures saturés et/ou insaturés et /ou aromatiques, de préférence des hydrocarbures saturés. Ledit effluent hydrocarbures est avantageusement constitué d'un mélange d'hydrocarbures ayant entre 6 et 40 atomes de carbone, de préférence entre 10 et 20 atomes de carbone. De manière non limitative, ledit effluent hydrocarbures peut être une coupe gazole ou kérosène désulfurée ou bien encore une coupe d'hydrocarbures produite par une unité de type Fischer-Tropsh.

L'ajout d'eau au sein de la section de lavage/contre-lavage, préférentielle, permet un meilleur fonctionnement du procédé d'élimination des impuretés et des huiles brunes selon l'invention.

Le procédé selon l'invention évite ainsi la purge régulière d'éthanol afin d'éviter l'accumulation des huiles brunes, ce qui permet d'améliorer les performances globales du procédé.

### Étape E) de traitement des effluents

Conformément à l'invention, l'étape E) de traitement des effluents est alimentée au moins par le raffinat éthanol/acétaldéhyde/eau issu de l'étape D) et produit au moins un effluent éthanol, un effluent acétaldéhyde et un effluent eau. Si l'effluent eau usée issu de l'étape F), ou l'effluent eau alcoolisée issu de l'étape C) n'ont pas subi l'étape D) d'élimination des impuretés et des huiles brunes, ils peuvent préférentiellementalimenter directement l'étape E) de traitement des effluents. La section E) est avantageusement également alimentée par une fraction de la charge éthanol.

De préférence et à la différence de l'art antérieur, aucun soutirage avec perte d'éthanol ou acétaldéhyde n'est effectué.

Ladite étape E) comprend au moins deux sections de distillation. Une section de distillation de l'eau et de l'éthanol, et une section de distillation de l'acétaldéhyde.

Selon un premier mode préférentiel , ledit raffinat éthanol/acétaldéhyde/eau issu de l'étape D) et éventuellement l'effluent eau usée issu de l'étape F) alimentent ladite section de distillation de l'acétaldéhyde, dans laquelle l'acétaldéhyde est séparé de manière à former un effluent acétaldéhyde, le résidu de ladite section de distillation de l'acétaldéhyde alimentant une section de distillation de l'eau et de l'éthanol permettant de séparer en tête un effluent éthanol et en fond un effluent eau. L'effluent eau alcoolisée issu de l'étape C) ne contenant pas d'acétaldéhyde, il alimente directement ladite section de distillation de l'eau et de l'éthanol. Ladite section de distillation de l'eau et de l'éthanol est également avantageusement alimentée par une fraction de la charge éthanol.

L'effluent éthanol issu de l'étape E) est de préférence constitué de plus de 80% poids d'éthanol, de préférence de plus de 84% poids. De manière non limitative, l'effluent éthanol issu de l'étape E) peut contenir des impuretés comme de l'eau, de l'acétate d'éthyle, du butanol et de l'hexanol. Les impuretés autre que l'eau représentent de préférence moins de 10%, de façon privilégiée moins de 5%, de façon encore préférentielle moins de 2% poids dudit effluent éthanol.

L'effluent acétaldéhyde issu de l'étape E) est préférentiellement constitué de plus de 80% pds d'acétaldéhyde et d'éthanol, de préférence de plus de 85 % poids. De manière non limitative, l'effluent acétaldéhyde issu de l'étape E) peut contenir des impuretés comme de l'eau, de l'acétate d'éthyle, de l'acétone. Les impuretés autre que l'eau représentent préférentiellement moins de 10%, de façon préférentielle moins de 5% poids dudit effluent acétaldéhyde.

Lesdits effluents acétaldéhyde, éthanol et eau sont ensuite recyclés dans le reste du procédé selon l'invention. La fraction dudit effluent acétaldéhyde alimentant l'étape A) est de préférence au moins de 0,60, de préférence au moins 0,65, de manière très préférée au moins 0,70. La fraction dudit effluent eau alimentant ladite étape C) est avantageusement compris entre 0 et 0,3, très avantageusement entre 0 et 0,1, plus avantageusement compris entre 0 et 0,05. La fraction dudit effluent eau alimentant ladite étape D) d'élimination des impuretés et des huiles brunes est avantageusement comprise entre 0 et 1, préférentiellement entre 0,3 et 0,6, et avantageusement entre 0,45 et 0,55.

Dans un autre mode préférentiel de réalisation de l'invention, lesdits effluents acétaldéhyde, éthanol et eau subissent une étape de purification avant d'être recyclés dans le reste du procédé. Par purification, on entend mettre en contact lesdits effluents avec des adsorbants comme par exemple du charbon actif, de la silice, de l'alumine ou encore une résine polymérique fonctionnalisée. Par exemple, un charbon actif permet d'éliminer les traces de butanol et d'hexanol comprises dans l'effluent éthanol. Par exemple, une résine basique permet d'éliminer l'acide acétique présent dans l'effluent eau. Quand les adsorbants sont saturés, et ne permettent pas de garantir la pureté des effluents acétaldéhyde, éthanol et eau, ils sont soit éliminés, soit régénérés pour être réutilisés.

### Etape F) de première purification du butadiène

L'étape F) de première purification du butadiène comprend au moins une section de lavage gaz-liquide alimentée en fond par l'effluent butadiène brut issu de B) et en tête par un flux d'eau pouvant être un flux d'eau d'origine externe audit procédé de production de butadiène et/ou une fraction de l'effluent eau issu de l'étape E), ladite section de lavage gaz-liquide produisant en tête un effluent butadiène pré-purifié et en fond un effluent eau usée. De manière préférée, ledit flux d'eau est un flux d'eau d'origine externe au procédé.

Ledit effluent eau usée contient de l'acétaldéhyde et un peu de butadiène, et est préférentiellement envoyé vers l'étape E) de traitement des effluents, vers la section de distillation de l'acétaldéhyde.

L'objectif de l'étape F) est d'éliminer les impuretés polaires, en particulier l'acétaldéhyde qui ne doit pas être présent au-delà de quelques ppm dans le butadiène final. L'effluent butadiène brut issu de B) comprend la majorité du butadiène, mais contient encore de nombreuses impuretés, dont une quantité importante d'acétaldéhyde qui forme un azéotrope avec le butadiène et ne peut donc pas être complètement éliminé par distillation lors de l'étape B). Ainsi, le débit dudit flux d'eau est ajusté pour obtenir la spécification recherchée en acétaldéhyde dans l'effluent butadiène pré-purifié.

Ledit flux d'eau est préférentiellement refroidi à une température inférieure à 25°C, de préférence inférieure à 20°C avant d'alimenter la section de lavage gaz-liquide de manière à faire le lavage avec une quantité d'eau réduite. La température d'alimentation dudit flux d'eau est choisie de manière à ne pas former d'hydrates avec le butadiène et les hydrocarbures légers encore présents dans le flux butadiène brut issu de l'étape B). Ladite section de lavage gaz-liquide est de préférence opérée à une pression telle que le butadiène est en phase gazeuse et non liquide. La pression sur cette section est comprise entre 0,1 et 1 MPa, et de manière préférée entre 0,2 et 0,3 MPa.

### Étape Fbis) optionnelle de seconde purification du butadiène

L'effluent butadiène pré-purifié issu de l'étape F) subit avantageusement une étape Fbis) de seconde purification du butadiène avant d'être alimentée dans l'étape G) ultérieure de purification du butadiène, ladite étape Fbis) comprenant au moins une section de lavage alimentée en fond par ledit effluent butadiène pré-purifié issu de F), et en tête par une solution absorbante. On soutire en tête de ladite section de lavage un effluent butadiène pré-purifié dans lequel ont été éliminées les traces d'acétaldéhyde encore contenues dans l'effluent butadiène pré-purifié, ainsi que les traces d'autres carbonyles qui sont moins solubles dans l'eau que l'acétaldéhyde, comme par exemple le butanal, l'acétone et l'hexanal, et donc moins efficacement éliminés par un simple lavage à l'eau. On soutire en fond de ladite section de lavage un effluent liquide qui est éliminé du procédé.

Dans un premier mode de réalisation de ladite étape Fbis), ladite solution absorbante est une solution aqueuse ayant un pH supérieur à 10, ajusté par l'ajout de soude ou de potasse.

Dans un deuxième mode de réalisation de ladite étape Fbis), ladite solution absorbante est une solution aqueuse de bisulfite de sodium ou de potassium dont le pH est compris entre 5 et 8, de manière préféré entre 6 et 7.

Dans un troisième mode de réalisation de ladite étape Fbis), ladite solution absorbante est une solution aqueuse contenant un composé de la famille des hydrazines.

La demanderesse a découvert que la combinaison des étapes F) et Fbis) était particulièrement bien adaptée au traitement d'un effluent butadiène brute issu d'un procédé de type Lebedev.

En effet, la mise en oeuvre de l'étape F) seule nécessite de gros débits d'eau pour atteindre la spécification de moins de 10 ppm de composés carbonylés. Ces gros débits d'eau sont ensuite traités à l'étape E), ce qui induit des coûts d'opération et d'investissement conséquents. De plus, en augmentant de manière très importante les débits d'eau alimentant l'étape F), on solubilise une faible portion de butadiène, ce qui diminue le rendement global du procédé.

Par ailleurs, la mise en oeuvre de l'étape Fbis) seule ne conviendrait pas pour traiter un effluent butadiène brut issu de l'étape B). En effet, l'acétaldéhyde éliminé de l'effluent butadiène brut par mise en contact avec une solution aqueuse basique ou une solution de bisulfite ou une solution aqueuse d'un composé de la famille des hydrazine, ne peut être facilement régénérée. Par conséquent, une quantité importante d'acétaldéhyde serait ainsi perdue, ce qui se traduirait par une baisse de rendement global du procédé.

La demanderesse a donc identifié un fonctionnement optimal et préférentiel du procédé en enchainant les étapes F) et Fbis) de première et seconde purification du butadiène, permettant respectivement d'atteindre les spécifications, tout en maximisant le rendement global du procédé et en minimisant les coûts opératoires.

### Étape G) ultérieure de purification du butadiène

Conformément à l'invention, une étape G) ultérieure de purification du butadiène est alimentée au moins par ledit effluent butadiène pré-purifié issu de ladite étape F), avantageusement traité dans l'étape Fbis) de seconde purification, et produit au moins un effluent butadiène purifié.

Cette étape G) permet de purifier le butadiène produit dans les étapes réactionnelles à un très haut niveau de pureté (typiquement plus de 99,5% poids, préférentiellement plus de 99,8% poids, et très préférentiellement plus de 99,9% poids), tout en limitant les pertes de produit en séparant les impuretés n'ayant pas ou partiellement été retirées au cours de l'étape B), F) et avantageusement Fbis).

Dans un premier mode de l'invention, ladite étape G) comprend au moins une section de séchage, une section de distillation cryogénique et une section de séparation butadiène/butènes par extraction liquide-liquide.

Selon ce mode de réalisation l'effluent butadiène pré-purifié issu de l'étape F), avantageusement traité dans l'étape Fbis), alimente une section de séchage. Cette section a pour objectif d'atteindre les spécifications requises en eau dans le produit final (effluent butadiène purifié), et de permettre de pratiquer une séparation cryogénique sans risque de formation d'hydrates. En sortie de ladite section de séchage, on obtient un effluent butadiène sec. Par butadiène sec on entend moins de 10 ppm d'eau, de préférence moins de 5 ppm, de préférence moins de 1 ppm.

Ladite section de séchage comprend préférentiellement un séchage constitué d'une ou plusieurs capacités contenant un ou plusieurs adsorbants ayant une forte affinité pour l'eau. De manière non limitative, cet adsorbant peut être constitué de silice et/ou d'alumine. De manière non limitative, cet adsorbant peut être une zéolite telle qu'une zéolite 3A ou 4A. Lorsque l'adsorbant ou les adsorbants sont saturés en eau, on alimente ledit effluent butadiène pré-purifié vers une autre capacité contenant l'adsorbant ou les adsorbants frais ou régénérés.

La régénération de l'adsorbant peut être réalisée soit en modifiant la pression partielle d'eau au sein de la capacité, soit en modifiant la température au sein de la capacité, soit en modifiant la pression partielle d'eau et la température au sein de la capacité. Dans ce dernier mode de réalisation, la régénération du ou des adsorbants saturé en eau est faite en chauffant la capacité, tout en l'alimentant avec un flux ne contenant pas, ou très peu, d'eau. Par pas ou très peu d'eau, on entend moins de 500 ppm, préférentiellement moins de 350 ppm, de manière préférée moins de 10 ppm, de préférence moins de 5 ppm, de manière très préférée moins de 1 ppm. Ce flux ne contenant pas ou très peu d'eau peut être de façon non limitative un flux d'azote, un flux d'air, un flux d'hydrocarbure, ou un flux d'hydrogène.

Ledit flux ne contenant pas ou très peu d'eau est chauffée à une température suffisante pour régénérer l'adsorbant ou les adsorbants avant d'être alimentée dans la capacité contenant l'adsorbant ou les adsorbants à régénérer, préférentiellement aux environs de 250°C.

Toujours selon ce mode de réalisation, ledit effluent butadiène sec alimente ensuite une section de distillation cryogénique mettant en oeuvre une colonne à distiller. Les produits légers sortent en tête de la section de distillation cryogénique entre -20°C et -35°C. Le fond de la colonne est à une température comprise entre 20 et 50°C, préférentiellement entre 25 et 45°C, de manière très préférée entre 30 et 40°C, la pression en tête de colonne est comprise entre 0,3 et 0,4 MPa, préférentiellement 0,35 MPa. L'intérêt de la colonne est de présenter une très grande efficacité de séparation des derniers incondensables et ceci sans perte de butadiène (moins de 0.05 %). On évite ainsi un recyclage important vers l'étape B) et une perte de butadiène.

Le produit de fond de ladite section de distillation cryogénique, appelé effluent butadiène étêté, comprend pour principale impureté des butènes. Ledit effluent butadiène étêté alimente une section de séparation butadiène/butènes par extraction liquide-liquide, tel que décrite dans le brevet FR 2,036,057.

Encore selon le premier mode de réalisation, ladite section de séparation butadiène/butènes est une section d'extraction liquide-liquide dans laquelle ledit effluent butadiène étêté alimente en une zone intermédiaire une première colonne d'extraction liquide-liquide, dans laquelle un flux de solvant polaire, préférentiellement du DMSO, est alimenté en tête. En fond, un solvant hydrocarbure saturé, préférentiellement du pentane ou du cyclohexane, est alimenté. Les débits ainsi que le ratio des débits de solvant polaire sur solvant hydrocarbure sont réglés de tel sorte que l'essentiel des butènes vont être entraînés par le solvant hydrocarbure et l'essentiel du butadiène être entraîné par le solvant polaire.

Le mélange butènes/hydrocarbure obtenu en tête de la première colonne d'extraction est ensuite traité dans une première colonne à distiller afin d'obtenir en tête l'effluent butènes, et en fond le solvant hydrocarbure qui peut être recyclé.

Le mélange butadiène/solvant polaire alimente ensuite la tête d'une deuxième colonne d'extraction liquide/liquide dans laquelle le butadiène est extrait du solvant polaire par mise en contact directe avec une quantité de solvant hydrocarbure plus importante que dans la première colonne d'extraction liquide-liquide, qui est introduite en fond de ladite deuxième colonne d'extraction liquide-liquide.

Le mélange butadiène/hydrocarbure obtenu en tête de la deuxième colonne d'extraction liquide-liquide est ensuite traité dans une colonne à distiller afin d'obtenir en tête l'effluent butadiène purifié, et en fond le solvant hydrocarbure qui peut être recyclé.

De manière préférée, les colonnes d'extraction liquide-liquide de ladite section de séparation butadiène/butènes sont opérées à une pression comprise entre 0,1 et 1 MPa, et une température comprise entre 20 et 60°C.

Dans un autre mode de l'invention, ladite étape G) comprend au moins une distillation et une distillation extractive. L'étape de distillation peut être réalisée en amont ou en aval de l'étape de distillation extractive. La distillation extractive peut être réalisée de manière non limitative avec un solvant comme la N-methyl-pyrrolidone, le dimethyl-formamide ou l'acétonitrile.

Les différentes étapes de traitement et de purification du butadiène B), F), Fbis) et G) peuvent bien évidemment également co-traiter tout flux comprenant du butadiène éventuellement produits par d'autres procédés situés à proximité du procédé selon l'invention.

### DESCRIPTION DES FIGURES

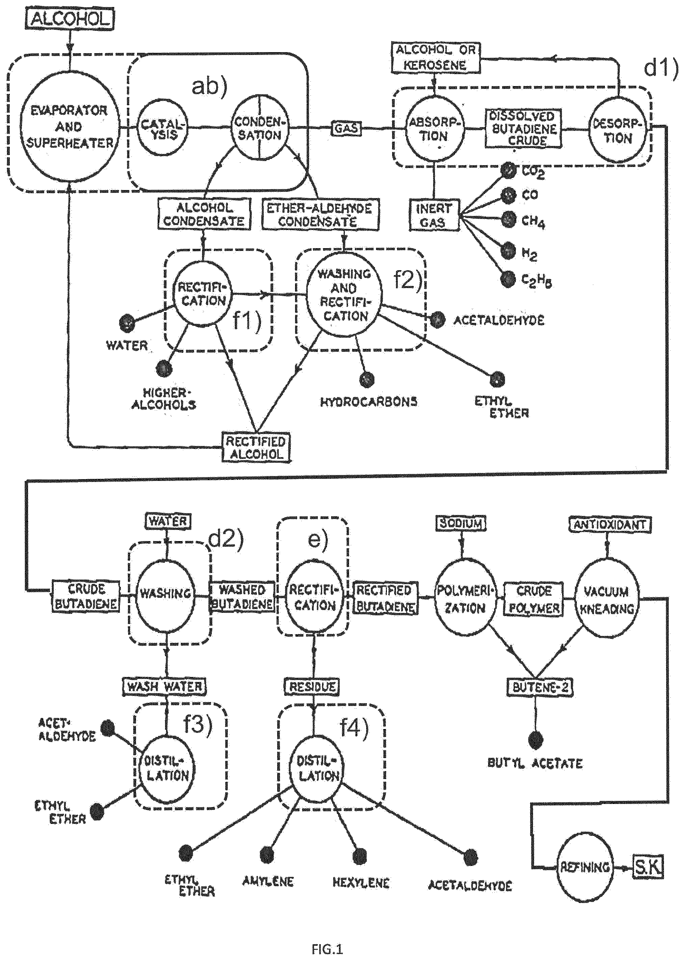

La **figure 1** représente de manière schématique un arrangement du procédé selon l'art antérieur, extrait de l'ouvrage « synthetic rubber from alcohol », Talalay et Magat, 1945.

Une étape ab) de conversion de l'éthanol en butadiène est alimentée par la charge éthanol, comprenant de l'éthanol et de l'eau, par l'effluent éthanol issu de l'étape f1) et par l'effluent acétaldéhyde issu de l'étape f2). L'étape ab) produit un effluent gaz, un effluent liquide léger et un effluent liquide lourd.

L'étape d1) d'extraction du butadiène comprend une section de lavage gaz-liquide par un solvant, et une section de régénération du solvant par distillation fonctionnant en boucle fermée. L'effluent gaz issu de l'étape ab) est introduit en fond de la colonne de lavage gaz-liquide, et le solvant est introduit en tête. La colonne de lavage gaz-liquide produit en tête l'effluent sous-produits gazeux et hydrogène, et en fond le solvant saturé en butadiène, qui est envoyée sur un plateau intermédiaire de la colonne à distiller. Ladite colonne à distiller produit en tête l'effluent butadiène brut, et en fond le solvant régénéré qui est recyclé vers la colonne de lavage gaz-liquide. Le solvant peut être un hydrocarbure comme par exemple de la turpidine, ou de l'éthanol. De manière régulière, le solvant de lavage est purgé, et la boucle est alimentée en solvant frais.

L'étape d2) de première purification du butadiène est alimentée par l'effluent butadiène brute issu de l'étape d1) et par un effluent eau et produit un effluent butadiène pré-purifié et un effluent eau usée. Cette étape comprend une section de lavage gaz-liquide, qui est alimentée en fond par l'effluent butadiène brute issu de d1) et en tête par ledit effluent eau. La colonne de lavage gaz-liquide produit en fond ledit effluent eau usée, et en tête ledit effluent butadiène pré-purifié.

L'étape e) ultérieure de purification du butadiène est alimentée par ledit effluent butadiène pré-purifié issu de ladite étape d2) et produit un effluent butadiène purifié et un résidu. Cette étape comprend une distillation.

L'étape f1) de traitement de effluent liquide lourd est alimentée par l'effluent liquide lourd issu de l'étape ab) et produit un effluent liquide léger, un effluent eau, un effluent éthanol, et un effluent alcools lourds qui est éliminé du procédé. Cette étape comprend au moins une distillation.

L'étape f2) de traitement de effluent liquide léger est alimentée par l'effluent liquide léger issu de l'étape ab) et par l'effluent liquide léger issu de l'étape f1). Elle produit un effluent acétaldéhyde, un effluent diéthyle éther et un effluent hydrocarbure et comprend au moins une distillation et une section de lavage.

L"étape f3) de traitement des eaux usées est alimentée par l'effluent eau usée issu de l'étape d2) et produit un effluent eau, un effluent acétaldéhyde, et un effluent diéthyle éther. Elle comprend au moins une distillation.

L'étape f4) de traitement du résidu est alimentée par le résidu issu de l'étape e) et produit un effluent acétaldéhyde, un effluent diéthyle éther, un effluent hydrocarbures C₅ et un effluent hydrocarbures C₆ qui sont éliminés du procédé. Elle comprend au moins une distillation.

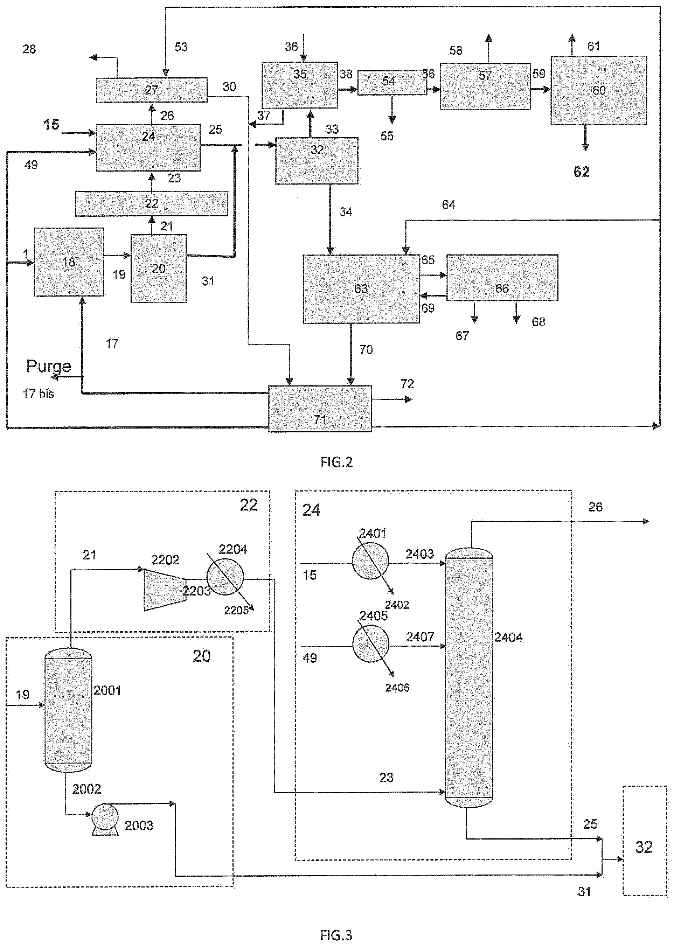

La **figure 2** représente de manière schématique et non limitative un arrangement du procédé selon l'invention.

Une fraction 1 de l'effluent éthanol issu de la section de distillation 71 est envoyé vers la section réactionnelle 18, ou une partie de l'éthanol est converti principalement en acétaldéhyde, butadiène et en hydrogène. La section réactionnelle 18 est par ailleurs alimentée par l'effluent acétaldéhyde 17 issu de la section de distillation 71. Le conduit 17bis permet d'ajuster le débit de l'effluent 17 alimentant la section réactionnelle 18. L'effluent de la section réactionnelle 18 est envoyé vers la section de séparation 20 par le conduit 19 pour être séparé en un effluent gazeux 21 et un effluent liquide 31.

L'effluent gazeux 21 est comprimé dans la section 22. Il alimente via le conduit 23 une section de lavage 24 dans laquelle il est lavé par mise en contact avec la charge éthanol 15 et l'éthanol recyclé 49. Cet ensemble est plus particulièrement décrit **figure 3**. L'effluent gazeux comprimé et lavé alimente par le conduit 26 une section de lavage à l'eau 27 dans laquelle il est lavé avec une fraction de l'effluent eau 53 issu de la section de distillation 71. L'eau chargée en éthanol après le lavage est renvoyée par le conduit 30 vers la section de distillation 71, directement dans la colonne de séparation eau-éthanol sans alourdir la colonne acétaldéhyde. L'effluent vapeur lavé dans la section 27 est soutiré par le conduit 28.

L'effluent liquide 31 issu du séparateur 20 est mélangée au liquide de fond de lavage 24 arrivant par le conduit 25. Le mélange est envoyé à la distillation 32, qui va séparer en tête une coupe butadiène 33 et en fond un mélange comprenant de eau, de l'éthanol, de l'acétaldéhyde et des impuretés. La coupe butadiène 33 est envoyée vers un lavage à l'eau 35 destiné à éliminer les impuretés polaires et spécialement l'acétaldéhyde. L'eau de lavage, qui est de l'eau propre, est introduite par le conduit 36. L'eau chargée en acétaldéhyde est renvoyée par le conduit 37 vers la section de distillation 71.

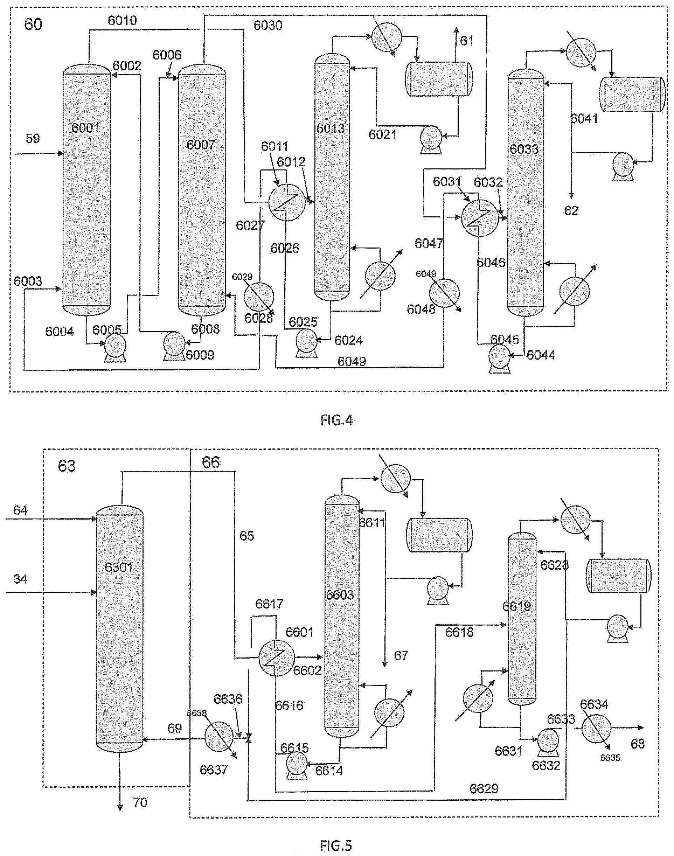

L'effluent butadiène pré-purifié est envoyé par le conduit 38 vers une section de séchage 54 afin d'éliminer toute trace d'eau. L'effluent butadiène sec 56 alimente une distillation cryogénique 57 et l'eau est évacuée par le conduit 55. Les produits légers sortent en tête de la section de distillation cryogénique à -35°C par la conduite 58, avec une perte très faible en butadiène. La coupe butadiène étêtée sort par le conduit 59 et arrive dans une section extraction liquide-liquide 60. Le fonctionnement de cette extraction est décrit plus en détail **figure 4****.**

L'effluent butadiène purifié sort de cette extraction par la conduite 62, à une pureté satisfaisante pour les spécifications actuelles (plus de 99.5%), les impuretés résiduelles étant principalement des butènes. Les butènes séparés dans cette section (comprenant une faible quantité de butadiène) sortent de l'unité par le conduit 61.

Le résidu éthanol/acétaldéhyde, produit de fond de la distillation 32, est envoyé par le conduit 34 vers la section 63 de lavage/contre-lavage aux hydrocarbures lourds 69 et à l'eau recyclée 64. Les hydrocarbures lourds de lavage, chargés en impuretés sortent par le conduit 65 et alimentent la section de régénération 66, dont on va sortir les hydrocarbures lourds 69 retournant au lavage, une fraction légère 67, contenant en particulier du di-éthyle éther et de l'éthyle acétate, plus quelques huiles brunes légères. On sort également une coupe lourde 68, contenant des huiles brunes lourdes et une petite partie des hydrocarbures de lavage. Le fonctionnement des sections 63 et 66 est décrit plus en détail **figure 5**.

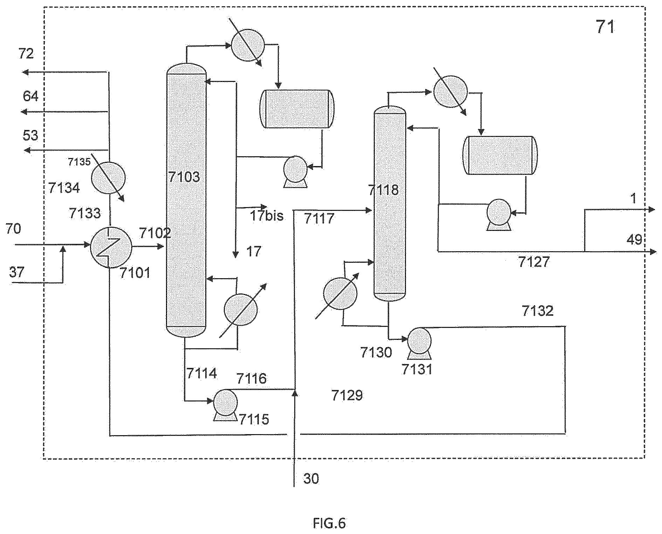

Le liquide de fond de la section de lavage/contre-lavage 63, contenant à la fois le résidu éthanol/acétaldéhyde 34 débarrassé de ses impuretés ayant de fortes affinités avec les hydrocarbures lourds 69 et l'eau de lavage 64, est envoyé par le conduit 70 vers la section de distillation 71. Cette section permet de séparer une fraction acétaldéhyde renvoyée par le conduit 17 vers la section réactionnelle 18, une fraction éthanol renvoyée en partie vers la section réactionnelle 18 par le conduit 1 et en partie vers le lavage 24 par le conduit 49, et une fraction eau, contenant un peu d'acide acétique, partiellement recyclée vers le lavage 27 par le conduit 53 et le lavage 63 par le conduit 64, le reste de l'eau étant purgée hors de l'unité par le conduit 72. Le fonctionnement de cette section est décrit en détails **figure 6**.

La **figure 3** représente de manière schématique et non limitative la séparation de l'effluent de la section réactionnelle 18 et une partie de l'étape de traitement de l'effluent butadiène.

L'effluent 19 de la section réactionnelle 18 alimente un séparateur 2001 dans lequel un effluent butadiène 21 et une phase liquide 2002 sont séparées. L'effluent butadiène 21 est comprimée dans un compresseur 2202, l'effluent vapeur comprimé 2203 étant ensuite refroidi dans un échangeur de chaleur 2204 par une utilité froide 2205.

L'effluent vapeur comprimé et refroidi 23 alimente la colonne de lavage adiabatique 2404, où il va être lavé par la charge éthanol 15 et le recyclage éthanol 49. La charge ethanol 15 est préalablement refroidie dans l'échangeur de chaleur 2401 par un produit réfrigérant arrivant par le conduit 2402. La charge éthanol préalablement refroidie arrive par le conduit 2403 dans la colonne 2404. Le recyclage éthanol 49 est préalablement refroidi dans l'échangeur de chaleur 2405 par un produit réfrigérant arrivant par le conduit 2406. La charge éthanol préalablement refroidie arrive par le conduit 2407 dans la colonne 2404. On récupère en tête de colonne un effluent vapeur comprimé et lavé 26 et en fond le liquide de fond de lavage 25.

Le liquide de fond de lavage 25 est mélangé avec la phase liquide 2002 qui a été au préalable pompée par la pompe 2003 et arrivant par le conduit 31. Le mélange de 25 et 31 contient la totalité du butadiène produit, et est envoyé vers la section 32.

La **figure 4** représente de manière schématique et non limitative la purification du butadiène à l'aide d'un solvant polaire, par exemple du DMSO (diméthyle sulfoxyde).

L'effluent butadiène sec alimente par le conduit 59 une première colonne d'extraction 6001, dans laquelle un flux de solvant polaire, qui peut être par exemple du DMSO, arrive en tête par le conduit 6002. En fond, un solvant hydrocarbure, tel qu'un pentane ou du cyclohexane est alimenté par le conduit 6003.

En fond de la colonne 6001, le solvant polaire et le butadiène dissous sortent par le conduit 6004, sont pompés par la pompe 6005, et sont envoyés en tête de la colonne 6007 par le conduit 6006. Une plus grande quantité de solvant hydrocarbure est injecté en fond de la colonne 6007 par le conduit 6049 afin de sortir le butadiène du solvant polaire. En fond de 6007, le solvant polaire débarrassé du butadiène sort par le conduit 6008 et est pompé par la pompe 6009 puis renvoyé vers la colonne 6001 par le conduit 6002.

En tête de colonne, le butadiène dissous dans les hydrocarbures est envoyé par le conduit 6030 vers l'échangeur de chaleur 6031, ou il est chauffé par échange indirect avec le fond de la colonne 6033. En sortie de l'échangeur de chaleur 6031, le mélange butadiène-solvant est alimenté dans la colonne 6033 par le conduit 6032.

En tête de colonne 6033, après condensation totale, une partie du liquide est envoyé comme reflux à la colonne 6033 par le conduit 6041. Le reste, qui constitue l'effluent butadiène purifié est envoyé hors du procédé par le conduit 62.

Le fond de la colonne 6033, qui est du solvant hydrocarbure, est envoyé par le conduit 6044 à la pompe 6045. En sortie de pompe 6045, le solvant est envoyé par le conduit 6046 vers l'échangeur de chaleur 6031, où il est refroidi par échange indirect de chaleur avec la charge de la colonne 6033. En sortie de l'échangeur de chaleur 6031, le solvant est envoyé par le conduit 6047 à l'échangeur de chaleur 6048, pour finir le refroidissement à l'aide d'une utilité froide 6049. En sortie de l'échangeur 6048, le solvant est renvoyé à la colonne de lavage 6007 par le conduit 6049.

En tête de la colonne 6001 sort un mélange solvant hydrocarbure et butènes, avec une petite perte en butadiène. Ce mélange est envoyé à l'échangeur de chaleur 6011, où il est chauffé par échange indirect avec le fond de la colonne 6013. En sortie de l'échangeur de chaleur 6011, le mélange butène-solvant alimente la colonne 6013 par le conduit 6012. En tête de colonne 6013, après condensation et séparation gaz/liquide, la phase liquide est envoyée comme reflux par le conduit 6021. La phase vapeur, constituée essentiellement de butènes et d'un peu de butadiène est sortie du procédé par le conduit 61 pour servir, par exemple, de combustible.

Le fond de la colonne 6013, qui est du solvant hydrocarbure, est envoyé par le conduit 6024 à la pompe 6025. En sortie de pompe 6025, le solvant est envoyé par le conduit 6026 à l'échangeur de chaleur 6011, où il est refroidi par échange indirect de chaleur avec la charge de la colonne 6013. En sortie de l'échangeur de chaleur 6011, le solvant est envoyé par le conduit 6027 à l'échangeur de chaleur 6028, pour finir le refroidissement à l'aide d'une utilité froide 6029. En sortie de l'échangeur 6028, le solvant est renvoyé à la colonne de lavage 6001 par le conduit 6003.

La **figure 5** représente de manière schématique et non limitative l'extraction des impuretés peu polaires et des huiles brunes par lavage/contre-lavage.

Le résidu éthanol/acétaldéhyde 34 alimente la colonne de lavage 6301. Le solvant hydrocarbures lourds (qui peut être par exemple du coupe gasoil ou kérosène désulfuré, ou une coupe produite par une unité type Fischer-Tropsh) est alimenté en fond de colonne 6301 par le conduit 69, tandis qu'une fraction de l'effluent eau 64 alimente la colonne 6301 en tête de colonne.

L'effluent hydrocarbures lourds de lavage est soutiré en tête de la colonne 6301 par le conduit 65 et est préchauffé dans l'échangeur de chaleur 6601 par échange avec le fond de la colonne 6603. À la sortie de l'échangeur 6601, L'effluent hydrocarbures lourds de lavage préchauffé 6602 est envoyé vers la colonne 6603.

En tête de colonne 6603, après condensation, une partie du liquide est envoyé comme reflux par le conduit 6611, le reste est envoyé hors de l'unité par le conduit 67.

Le fond de la colonne 6603 est envoyé par le conduit 6614 à la pompe 6615. Une fraction du liquide en sortie de pompe 6615 est envoyé par le conduit 6618 vers une autre colonne à distiller 6619. La fraction restante est envoyée par le conduit 6616 vers l'échangeur de chaleur 6601, qui va permettre de refroidir le liquide de fond 6616 par échange indirect avec la charge 65 de la colonne 6603. Le liquide de fond refroidi sort de l'échangeur 6601 par le conduit 6617 pour être envoyé à l'échangeur de chaleur 6637.

En tête de colonne 6619, après condensation, une partie du liquide est envoyé comme reflux à la colonne 6619 par le conduit 6628, le reste est envoyé par le conduit 6629 vers l'échangeur 6637 en mélange avec le fond de la colonne 6603 où il est refroidi à l'aide d'une utilité froide 6638. Les hydrocarbures en sortie de 6637 sont renvoyés par le conduit 69 vers la colonne de lavage 6301.

La tête de colonne 6619 est composée pour l'essentiel d'hydrocarbures lourds avec encore quelques traces d'"huile noire". Un appoint équivalent d'hydrocarbures lourds (non représenté) est nécessaire pour maintenir le débit de lavage constant.

Le liquide de fond de la colonne 6619 sort par le conduit 6631 et est pompé par la pompe 6632 puis envoyé par le conduit 6633 vers un échangeur de chaleur 6634, ou il est refroidi par une utilité froide 6635, pour être sorti du procédé par le conduit 68. Il pourra ensuite servir, par exemple, de combustible. Le fluide 6635 peut être un flux de l'unité devant être réchauffé.

La **figure 6** présente un arrangement possible pour la section de distillation 71.

Le mélange éthanol/acétaldéhyde/eau/impuretés polaires issu du lavage 63 est amené par le conduit 70 vers la section de distillation 71. Ce flux est mélangé avec l'eau de lavage chargé en acétaldéhyde arrivant par le conduit 37 de la section de lavage 35. Le mélange de ces deux flux est chauffé par échange thermique indirect contre le flux 7133 dans l'échangeur de chaleur 7101. La sortie de cet échangeur est amenée à la colonne 7103 par le conduit 7102. La tête de la colonne est totalement condensée et un distillat (effluent riche en acétaldéhyde) est envoyé par le conduit 17 vers la section réactionnelle 18. Une fraction du distillat est purgé par le conduit 17bis. L'ajustement du ratio entre ces deux destinations permet d'ajuster le ratio Ethanol/acétaldéhyde à l'entrée du réacteur de la section 18. Ce distillat comporte principalement de l'acétaldéhyde, mais aussi de l'eau, de l'éthanol et d'autres impuretés légères (diéthyl éther, butanal, acétone, éthyl acétate, etc).

Le produit de fond de la colonne 7103, contenant principalement de l'eau, de l'éthanol, un peu de butanol, de l'acide acétique et quelques autres impuretés sort par le conduit 7114, puis est envoyé à l'aide de la pompe 7115 par le conduit 7116 vers la colonne 7118. L'eau de lavage de la section de lavage 27, chargée en éthanol arrive par le conduit 30 et est mélangée avec le produit de fond de 7103 arrivant par le conduit 7116. Le mélange est envoyé par le conduit 7117 vers la colonne 7118.

La tête de la colonne 7118 est totalement condensée et un distillat est envoyé par le conduit 7127, en partie vers la section réactionnelle 18 par le conduit 1, l'autre partie vers le lavage24 par le conduit 49 (effluent riche en éthanol).. Ce distillat comporte principalement de l'éthanol, mais également de l'eau, un peu de butanol, et quelques autres impuretés.

Le produit de fond de la colonne 7118, (effluent riche en eau) contenant principalement de l'eau, et un peu d'acide acétique sort par le conduit 7130 et est envoyé à l'aide de la pompe 7131 par le conduit 7132 vers l'échangeur de chaleur 7101 où il est refroidi par échange indirect avec la charge de la colonne 7103. Le produit sort de 7101 par le conduit 7133 et est refroidi dans l'échangeur 7134 à l'aide d'une utilité froide 7135. A la sortie de l'échangeur, une partie de l'eau est envoyé par le conduit 64 vers le lavage 63 et par le conduit 53 vers le lavage 27, le reste est purgé par le conduit 72 à l'extérieur de l'unité.

### EXEMPLE - Procédé de production de butadiène selon l'invention

L'exemple suivant est basé sur des simulations procédés prenant en compte les recyclages des flux, et intégrant des données thermodynamiques calées sur des points expérimentaux (données d'équilibre liquide-vapeur binaires et coefficient de partage liquide-liquide). Le débit de charge est ajusté de manière à obtenir une production annuelle de 150 kt/an d'un butadiène ayant une pureté comprise entre 99,5 et 100% poids (en adéquation avec l'utilisation actuelle du produit), avec une durée de fonctionnement annuelle du procédé de 8000 h. Nous détaillons ci-dessous le fonctionnement de certaines étapes du procédé selon l'invention.

### 1.1 - Étape d'extraction du butadiène, alternative 1

Dans cet arrangement, l'effluent vapeur de l'étape de conversion en butadiène est comprimé, puis lavé par mise en contact avec la charge éthanol du procédé de conversion et par de l'éthanol recyclé. L'effluent gaz en sortie de lavage forme l'effluent gaz lavé.

Ladite charge éthanol a la composition suivante : 93,3% poids en éthanol et 6,7 % d'eau, sans trace mesurable d'impuretés; elle alimente la colonne de lavage à une température de 14°C, le débit de la charge est de 63 T/h. Le deuxième flux alimentant la colonne de lavage est un flux d'éthanol recyclé (contenant 84 % d'éthanol, le reste étant de l'eau), est refroidi également à 14°C, le débit de ce flux est de 130 T/h. Ce lavage permet de récupérer 99.93 % du butadiène compris dans ledit effluent vapeur. L'effluent gaz lavé est exempt d'acétaldéhyde, contrairement aux exemples selon l'art antérieur.

L'effluent gaz lavé est ensuite lavé avec de l'eau recyclée pour récupérer l'éthanol. Il est nécessaire d'employer 14 t/h d'eau pour récupérer l'ensemble de l'éthanol.

### 1.2 - Étape d'extraction du butadiène, alternative 2

Cet arrangement se différencie du précédent en ce que la charge éthanol et le flux de recyclage éthanol sont préalablement refroidis à -10°C avant d'être utilisés pour le lavage de l'effluent vapeur de l'étape de conversion en butadiène préalablement comprimé. Dans cette variante, le débit de charge est identique, en revanche le débit d'éthanol recyclé est de 78 T/h. En effet, la séparation étant plus efficace à température plus basse, le débit d'éthanol recyclé est réduit, passant de 130 à 78 T/h. Ce lavage permet de récupérer la totalité du butadiène (100%) compris dans ledit effluent vapeur.

L'effluent gaz lavé, exempt d'acétaldéhyde, est ensuite lavé avec de l'eau recyclée pour récupérer l'éthanol. Il est nécessaire d'employer 3.7 t/h d'eau pour récupérer l'ensemble de de l'éthanol.

Le refroidissement de la charge éthanol et du recyclage permet la diminution du débit d'éthanol recyclé, réduisant la charge dans tous les équipements du schéma procédé. De plus le débit d'eau requis pour le lavage est également réduit (divisé par 3.8), ce qui permet de réduire de 11% le débit d'eau en entrée de l'étape de traitement des effluents, réduisant par conséquent la taille des équipements de séparation et leur consommation énergétique.

### 1.3 - Étape d'extraction du butadiène, alternative 3

Dans cet arrangement, l'effluent vapeur de l'étape de conversion en butadiène est comprimé, puis lavé par mise en contact avec uniquement un effluent éthanol issu de l'étape de traitement des effluents. L'effluent gaz en sortie de lavage forme l'effluent gaz lavé.

Ledit effluent éthanol issu de l'étape de traitement des effluents a la composition suivante : 84% poids en éthanol et 16 % d'eau, sans trace mesurable d'impuretés. Il alimente la colonne de lavage à une température de -10°C et a un débit de 207 T/h. Ce lavage permet de récupérer la totalité du butadiène compris dans ledit effluent vapeur. L'effluent gaz lavé est exempt d'acétaldéhyde, contrairement à ce qui est le cas dans l'art antérieur.

L'effluent gaz lavé est ensuite lavé avec de l'eau recyclée pour récupérer l'éthanol. Il est nécessaire d'employer 8.1 t/h d'eau pour récupérer l'ensemble de de l'éthanol.

Cet arrangement est à comparer avec l'arrangement 1.2 précédent. On note que l'invention mise en oeuvre suivant l'arrangement 1.2, en utilisant la charge éthanol et un complément d'effluent éthanol recyclé, permet de réduire le débit de flux éthanol lavant la coupe butadiène de 8% par rapport à l'emploi uniquement d'un effluent éthanol issu de l'étape de traitement des effluents. Ce gain se traduit directement par des investissements et coûts opératoires plus faibles. De plus le débit d'eau nécessaire à la récupération du butadiène est divisé par 2, ce qui va soulager les colonnes à distiller en cout d'investissement et opératoires.

### 1.4 - Étape G) ultérieure de purification du butadiène

L'effluent butadiène purifié issu de l'étape de première purification du butadiène alimente une section de séchage, par passage successif sur une alumine, puis sur une zéolite 4A, afin d'abattre la totalité de l'eau éventuellement présente dans ledit effluent butadiène purifié. L'effluent de la section de séchage forme l'effluent butadiène sec. Cet effluent butadiène sec alimente ensuite une colonne de distillation cryogénique, opérée avec une pression en tête de 0,35 Mpa, une température de fond de 35°C et une température de tête de -23°C.

Le résidu de distillation, appelé effluent butadiène étêté, alimente enfin une section d'extraction liquide-liquide mettant en oeuvre du DMSO et du cyclohexane.

Une première colonne de lavage est alimentée en tête par 252 t/h de DMSO et en fond par 50 t/h de cyclohexane. Cette première colonne comprend 20 étages théoriques de lavage. Le produit de fond de la première colonne est envoyé vers une deuxième colonne de lavage comprenant 10 étages théoriques.

Le produit de tête de cette seconde colonne de lavage est traité dans une colonne à distiller, qui permet de séparer le butadiène du cyclohexane, comprenant 24 étages théoriques et opérée avec un taux de reflux de 8.3.

Le produit de tête de la première colonne de lavage est traité dans une colonne à distiller, qui permet de séparer les butènes du cyclohexane, comprenant 26 étages théoriques et opérée avec un taux de reflux de 10.

99.68% du butadiène entrant dans la l'étape de seconde séparation du butadiène est récupéré en tant que produit, avec une pureté de 99.68% poids.

On obtient en sortie de section d'extraction liquide-liquide un effluent butadiène purifié dont la teneur en butadiène est de 99,68% poids. Les pertes en butadiène sur l'ensemble des étapes de purification (calculées à partir du ratio du débit de butadiène pur compris dans l'effluent butadiène purifié sur le débit de butadiène pur compris dans l'effluent du réacteur de conversion en butadiène) est inférieur à 0,7 % poids.

L'agencement des étapes et des recyclages selon l'invention, en particulier en évitant l'accumulation d'impuretés, permet de recycler la quasi-totalité des composés n'ayant pas réagi. Ainsi, malgré une conversion par passe dans les réacteurs faible, et comparable à l'art antérieur, le rendement global est amélioré de plus de 10 points par rapport à l'art antérieur avec un rendement t_{butadiène} produit par t_{éthanol converti} de 36,8% et une récupération et une valorisation de 99,9% de l'éthanol compris dans la charge du procédé.

## Revendications

1. Procédé de production de butadiène à partir d'une charge éthanol comprenant au moins 80% poids d'éthanol comprenant au moins :
A) une étape de conversion de l'éthanol en butadiène comprenant au moins une section réactionnelle, alimentée au moins par l'effluent éthanol et une fraction de l'effluent acétaldéhyde issus de l'étape E), opérée à une pression comprise entre 0,1 et 1,0 MPa et à une température comprise entre 300 et 400°C en présence d'un catalyseur, et une section de séparation permettant de séparer l'effluent de ladite section réactionnelle en au moins un effluent gazeux et un effluent liquide, la fraction de l'effluent acétaldéhyde issu de l'étape E) n'alimentant pas ladite section réactionnelle constituant le flux de purge ;
B) une étape d'extraction du butadiène comprenant au moins une section de compression comprimant ledit effluent gazeux issu de l'étape A) à une pression comprise entre 0,1 et 1,0 MPa, une section de lavage gaz-liquide alimentée par un flux éthanol constitué de ladite charge éthanol du procédé et/ou une fraction de l'effluent éthanol issu de l'étape E), et par ledit effluent gazeux comprimé, dans laquelle l'alimentation gaz est réalisée à une température comprise entre 10 et 60°C, et l'alimentation liquide est réalisée à une température comprise entre 20°C et - 30°C, et produisant au moins un flux éthanol enrichi en butadiène et un effluent sous-produits gazeux, et une section de distillation alimentée par ledit flux éthanol enrichi en butadiène et produisant un effluent butadiène brut et un résidu éthanol/acétaldéhyde/eau, ladite section de distillation étant opérée entre 0,1 et 1 MPa ;
C) une étape de lavage à l'eau des sous-produits gazeux, alimentée par l'effluent sous-produits gazeux issu de l'étape B), ainsi que par une fraction de l'effluent riche en eau issu de ladite étape E) et produisant au moins un effluent eau alcoolisée ;
D) une étape d'élimination des impuretés et des huiles brunes, alimentée au moins par l'effluent éthanol/acétaldéhyde/eau issu de l'étape B), et par une fraction de l'effluent eau issu de l'étape E), et produisant au moins un raffinat eau / éthanol / acétaldéhyde, un effluent huiles brunes légères et un effluent huiles brunes lourdes ;
E) une étape traitement des effluents alimentée au moins par le raffinat eau / éthanol / acétaldéhyde issu de l'étape D), et produisant au moins un effluent éthanol, un effluent acétaldéhyde et un effluent eau ;
F) une étape de première purification du butadiène comprenant au moins une section de lavage gaz-liquide alimentée en fond par l'effluent butadiène brut issu de B) et en tête par un flux d'eau pouvant être un flux d'eau d'origine externe audit procédé de production de butadiène et/ou une fraction de l'effluent eau issu de l'étape E), ladite section de lavage produisant en tête un effluent butadiène pré-purifié et en fond un effluent eau usée ;
G) une étape ultérieure de purification du butadiène, alimentée au moins par ledit effluent butadiène pré-purifié issu de ladite étape F), et produisant au moins un effluent butadiène purifié.

2. Procédé selon la revendication 1 dans lequel l'effluent butadiène pré-purifié issu de l'étape F) subit étape Fbis) de seconde purification du butadiène avant d'être alimentée dans l'étape G) ultérieure de purification du butadiène, ladite étape Fbis) comprenant au moins une section de lavage alimentée en fond par ledit effluent butadiène pré-purifié issu de F), et en tête par une solution absorbante.

3. Procédé selon la revendication 2 dans lequel ladite solution absorbante est une solution aqueuse ayant un pH supérieur à 10, ajusté par l'ajout de soude ou de potasse.

4. Procédé selon la revendication 2 dans lequel ladite solution absorbante est une solution aqueuse de bisulfite de sodium ou de potassium dont le pH est compris entre 5 et 8.

5. Procédé selon la revendication 2 dans lequel ladite solution absorbante est une solution aqueuse contenant un composé de la famille des hydrazines.

6. Procédé selon l'une des revendications 1 à 5 dans lequel l'étape D) comprend au moins une section de lavage/contre-lavage, une section de distillation des huiles brunes légères, et une section de distillation des huiles brunes lourdes, ladite section de lavage/contre-lavage étant alimentée en un point intermédiaire par ledit effluent éthanol/acétaldéhyde/eau issu de l'étape B), en fond par un effluent hydrocarbures et en tête par une fraction de l'effluent eau issue de l'étape E) et produisant au moins un extrait hydrocarbures de lavages et un raffinat éthanol/acétaldéhyde/eau, ladite section de distillation des huiles brunes légères étant alimentée par ledit extrait hydrocarbures de lavages et produisant en tant que distillat un effluent huiles brunes légères, et un résidu hydrocarbures, ladite section de distillation des huiles lourdes étant alimentée par une fraction comprise entre 5 et 30% du débit total dudit résidu hydrocarbures et produisant un distillat hydrocarbures et, en tant que résidu, un effluent huiles brunes lourdes, ledit distillat hydrocarbures et la fraction non traitée dudit résidu hydrocarbures étant mélangés afin de constituer l'effluent hydrocarbures alimentant ladite section de lavage/contre-lavage.

7. Procédé selon l'une des revendications 1 à 6 dans lequel ladite section réactionnelle de ladite étape A) est également alimentée par une fraction de ladite charge éthanol.

8. Procédé selon l'une des revendications 1 à 7 dans lequel ladite étape A) est également alimentée par un flux externe d'acétaldéhyde.

9. Procédé selon l'une des revendications 1 à 8 dans lequel le flux de purge de ladite étape A) est transformé dans une étape d'hydrogénation dédiée pour produire de l'éthanol qui sera alors envoyé vers la dite section réactionnelle de ladite étape A).

10. Procédé selon l'une des revendications 1 à 9 dans lequel le flux éthanol enrichi en butadiène soutiré en fond de ladite section de lavage gaz-liquide de l'étape B) est mélangé à l'effluent liquide issu de l'étape A) de manière à former l'alimentation de ladite section de distillation de l'étape B).

11. Procédé selon l'une des revendications 1 à 9 dans lequel le flux éthanol enrichi en butadiène soutiré en fond de ladite section de lavage gaz-liquide de l'étape B) constitue l'alimentation de ladite section de distillation de l'étape B), l'effluent liquide issu de l'étape A) alimentant alors directement l'étape D) d'élimination des impuretés liquides et des huiles brunes.

12. Procédé selon la revendication 6 dans lequel ladite section de lavage/contre-lavage de ladite étape D), est alimentée en un point intermédiaire par ledit effluent éthanol/acétaldéhyde/eau issu de l'étape B), en mélange avec l'effluent eau usée issu de l'étape E), l'effluent eau alcoolisée issu de l'étape C) et/ou en mélange avec une fraction de l'effluent eau usée issu de l'étape F).

13. Procédé selon l'une des revendications 1 à 12 dans lequel ladite section E) est également alimentée par une fraction de la charge éthanol.

14. Procédé selon l'une des revendications 1 à 13 dans lequel ladite étape G) comprend au moins une section de séchage, une section de distillation cryogénique et une section de séparation butadiène/butènes par extraction liquide-liquide.

15. Procédé selon l'une des revendications 1 à 14 dans lequel ladite étape G) comprend au moins une distillation et une distillation extractive.

## Patentansprüche

1. Verfahren zur Herstellung von Butadien aus einem Ethanoleinsatz, umfassend mindestens 80 Gew.-% Ethanol, umfassend mindestens:
A) einen Schritt zum Umwandeln des Ethanols in Butadien, umfassend mindestens einen Reaktionsabschnitt, der mindestens mit dem Ethanolabstrom und einer Fraktion des aus Schritt E), erhaltenen Acetaldehydabstroms gespeist wird, der bei einem Druck im Bereich zwischen 0,1 und 1,0 MPa und bei einer Temperatur im Bereich zwischen 300 und 400 °C in Gegenwart eines Katalysators arbeitet, und einen Trennabschnitt zum Trennen des Abstroms des Reaktionsabschnitts in mindestens einen gasförmigen Abstrom und einen flüssigen Abstrom, wobei die aus Schritt E) erhaltene Fraktion des Acetaldehydabstroms, die den Reaktionsabschnitt nicht speist, den Spülstrom bildet;
B) einen Schritt zum Extrahieren von Butadien, umfassend mindestens einen Verdichtungsabschnitt, der den aus Schritt A) erhaltenen gasförmigen Abstrom bei einen Druck im Bereich zwischen 0,1 und 1,0 MPa verdichtet, einen Gas-Flüssigkeits-Waschabschnitt, der mit einem Ethanolstrom gespeist wird, der aus dem Ethanoleinsatz des Verfahrens und/oder einer Fraktion des aus Schritt E) erhaltenen Ethanolabstroms und aus dem verdichteten gasförmigen Abstrom besteht, wobei die Gaseinspeisung bei einer Temperatur im Bereich zwischen 10 und 60 °C ausgeführt wird, und die Flüssigkeitseinspeisung bei einer Temperatur im Bereich zwischen 20 °C und -30 °C ausgeführt wird, und der mindestens einen mit Butadien angereicherten Ethanolstrom und einen gasförmigen Nebenproduktabstrom herstellt, und einen Destillationsabschnitt, der mit dem mit Butadien angereicherten Ethanolstrom gespeist wird und einen unraffinierten Butadienabstrom und einen Ethanol/Acetaldehyd/Wasser-Rückstand herstellt, wobei der Destillationsabschnitt zwischen 0,1 und 1 MPa arbeitet;
C) einen Schritt zum Waschen mit Wasser der gasförmigen Nebenprodukte, der mit dem aus Schritt B) erhaltenen Abstrom von gasförmigen Nebenprodukten sowie mit einer Fraktion des aus Schritt E) erhaltenen wasserreichen Abstroms gespeist wird und der mindestens einen Abstrom aus alkoholhaltigem Wasser herstellt;
D) einen Schritt zum Entfernen von Verunreinigungen und braunen Ölen, der mit mindestens dem aus Schritt B) erhaltenen Ethanol/Acetaldehyd/Wasser-Abstrom und mit einer aus Schritt E) erhaltenen Fraktion des Abstroms gespeist wird, und der mindestens ein Wasser/Ethanol/Acetaldehyd-Raffinat, einen Abstrom brauner Leichtöle und einen Abstrom brauner Schweröle herstellt;
E) einen Schritt zum Behandeln der Abströme, der mit mindestens dem aus Schritt D) erhaltenen Wasser/Ethanol/Acetaldehyd-Raffinat gespeist wird, und der mindestens einen Ethanolabstrom, einen Acetaldehydabstrom und einen Wasserabstrom herstellt;
F) einen Schritt zum ersten Reinigen des Butadiens, umfassend mindestens einen Gas-Flüssigkeits-Waschabschnitt, der am Boden mit dem aus Schritt B) erhaltenen unraffinierten Butadienabstrom und am Kopf mit einem Wasserstrom gespeist wird, der ein Wasserstrom mit einem Ursprung außerhalb des Verfahrens zur Herstellung von Butadien und/oder eine Fraktion des aus Schritt E) erhaltenen Abstroms sein kann, wobei der Waschabschnitt am Kopf einen vorgereinigten Butadienabstrom und am Boden einen Abstrom aus gebrauchtem Wasser herstellt;
G) einen anschließenden Schritt zum Reinigen des Butadiens, der mit mindestens dem aus dem Schritt F) erhaltenen, vorgereinigten Butadienabstrom gespeist wird und der mindestens einen gereinigten Butadienabstrom herstellt.

2. Verfahren nach Anspruch 1, wobei der aus Schritt F) erhaltene vorgereinigte Butadienabstrom Schritt Fa) zum zweiten Reinigen des Butadiens unterzogen wird, bevor er nach dem Reinigen des Butadiens in Schritt G) gespeist wird, wobei Schritt Fa) mindestens einen Waschabschnitt umfasst, der am Boden mit dem aus Schritt F) erhaltenen vorgereinigten Butadienabstrom und am Kopf mit einer Absorptionslösung gespeist wird.

3. Verfahren nach Anspruch 2, wobei die Absorptionslösung eine wässrige Lösung mit einem pH-Wert von mehr als 10 ist, die durch Zugabe von Natrium- oder Kaliumhydroxid eingestellt wird.

4. Verfahren nach Anspruch 2, wobei die Absorptionslösung eine wässrige Natrium- oder Kaliumbisulfitlösung ist, deren pH-Wert im Bereich zwischen 5 und 8 liegt.

5. Verfahren nach Anspruch 2, wobei die Absorptionslösung eine wässrige Lösung ist, die eine Verbindung aus der Hydrazin-Familie enthält.

6. Verfahren nach einem der Ansprüche 1 bis 5, wobei Schritt D) mindestens einen Wasch-/Rückwaschabschnitt, einen Abschnitt zum Destillieren von braunen Leichtölen und einen Abschnitt zum Destillieren von braunen Schwerölen umfasst, wobei der Wasch-/Rückwaschabschnitt an einem Zwischenpunkt mit dem aus Schritt B) erhaltenen Ethanol/Acetaldehyd/Wasser-Abstrom, am Boden mit einem Kohlenwasserstoffabstrom und am Kopf mit einer Fraktion des aus Schritt E) erhaltenen Wasserabstroms gespeist wird und mindestens einen Kohlenwasserstoffextrakt-Waschextrakt und ein Ethanol/Acetaldehyd/Wasser-Raffinat herstellt, wobei der Abschnitt zum Destillieren der braunen Leichtöle mit dem Kohlenwasserstoff-Waschextrakt gespeist wird und als Destillat einen Abstrom brauner Leichtöle und einen Kohlenwasserstoff-Rückstand herstellt, wobei der Abschnitt zum Destillieren der Schweröle mit einer Fraktion im Bereich zwischen 5 und 30 % des gesamten Stromvolumens des Kohlenwasserstoff-Rückstands und einem Kohlenwasserstoffdestillat gespeist wird und als Rückstand einen Abstrom brauner Schweröle herstellt, wobei das Kohlenwasserstoffdestillat und die nicht behandelte Fraktion des Kohlenwasserstoff-Rückstands gemischt werden, um den Kohlenwasserstoffabstrom zu bilden, der den Wasch-/Rückwaschabschnitt speist.

7. Verfahren nach einem der Ansprüche 1 bis 6, wobei der Reaktionsabschnitt des Schritts A) ebenfalls mit einer Fraktion des Ethanoleinsatzes gespeist wird.

8. Verfahren nach einem der Ansprüche 1 bis 7, wobei der Schritt A) ebenfalls mit einem externen Acetaldehydstrom gespeist wird.

9. Verfahren nach einem der Ansprüche 1 bis 8, wobei der Spülstrom aus dem Schritt A) in einem Hydrierungsschritt umgewandelt wird, um Ethanol herzustellen, das dann zum Reaktionsabschnitt des Schritts A) geschickt wird.

10. Verfahren nach einem der Ansprüche 1 bis 9, wobei der mit Butadien angereicherte Ethanolstrom, der vom Boden des Gas-Flüssigkeits-Waschabschnittes aus Schritt B) abgezogen wird, mit dem aus Schritt A) erhaltenen flüssigen Abstrom derart vermischt wird, dass er die Speisung des Destillationsabschnitts des Schritts B) bildet.

11. Verfahren nach einem der Ansprüche 1 bis 9, wobei der mit Butadien angereicherte Ethanolstrom, der am Boden des Gas-Flüssigkeits-Waschabschnitt von Schritt B) abgezogen wird, die Speisung des Destillationsabschnitts aus Schritt B) bildet, wobei der aus Schritt A) erhaltene flüssige Abstrom dann direkt Schritt D) zum Entfernen der flüssigen Verunreinigungen und der braunen Öle speist.

12. Verfahren nach Anspruch 6, wobei der Wasch-/Rückwaschabschnitt des Schrittes D) mit dem aus Schritt B) erhaltenen Ethanol/Acetaldehyd/Wasser-Abstrom an einem Zwischenpunkt im Gemisch mit dem aus Schritt E) erhaltenen Abstrom des gebrauchten Wassers, dem aus Schritt C) erhaltene Abstrom aus alkoholhaltigem Wasser und/oder im Gemisch mit einer aus Schritt F) erhaltenen Fraktion des Abstroms aus gebrauchtem Wasser gespeist wird.

13. Verfahren nach einem der Ansprüche 1 bis 12, wobei der Abschnitt E) ebenfalls mit einer Fraktion des Ethanoleinsatzes gespeist wird.

14. Verfahren nach einem der Ansprüche 1 bis 13, wobei der Schritt G) mindestens einen Trocknungsabschnitt, einen Tieftemperaturdestillationsabschnitt und einen Abschnitt zur Butadien/Buten-Trennung durch Flüssig-Flüssig-Extraktion umfasst.

15. Verfahren nach einem der Ansprüche 1 bis 14, wobei der Schritt G) mindestens eine Destillation und eine Extraktivdestillation umfasst.

## Claims

1. A process for the production of butadiene from an ethanol feed comprising at least 80% by weight of ethanol, comprising at least:
A) a step for converting the ethanol into butadiene, comprising at least one reaction section supplied with at least the ethanol effluent and a fraction of the acetaldehyde effluent obtained from step E), operated at a pressure in the range 0.1 to 1.0 MPa and at a temperature in the range 300°C to 400°C in the presence of a catalyst, and a separation section for separating the effluent from said reaction section into at least a gaseous effluent and a liquid effluent, the fraction of the acetaldehyde effluent obtained from step E) which is not supplied to said reaction section constituting the purge stream;
B) a step for extracting butadiene, comprising at least one compression section compressing said gaseous effluent obtained from step A) to a pressure in the range 0.1 to 1.0 MPa, a gas-liquid scrubbing section supplied with an ethanol stream constituted by said ethanol feed for the process and/or a fraction of the ethanol effluent obtained from step E) and with said compressed gaseous effluent, in which the gas is supplied at a temperature in the range 10°C to 60°C, and the liquid is supplied at a temperature in the range 20°C to -30°C, and producing at least one ethanol stream enriched in butadiene and a gaseous effluent of by-products, and a distillation section supplied with said ethanol stream enriched in butadiene and producing an unrefined butadiene effluent and an ethanol/acetaldehyde/water residue, said distillation section being operated at between 0.1 and 1 MPa;
C) a step for scrubbing gaseous by-products with water supplied with the effluent of gaseous by-products obtained from step B), as well as with a fraction of the water-rich effluent obtained from said step E) and producing at least one alcohol-containing water effluent;
D) a step for eliminating impurities and brown oils, supplied with at least the ethanol/acetaldehyde/water effluent obtained from step B), and with a fraction of the water effluent obtained from step E), and producing at least one water/ethanol/acetaldehyde raffinate, a light brown oil effluent and a heavy brown oil effluent;
E) an effluent treatment step supplied with at least the water/ethanol/acetaldehyde raffinate obtained from step D), and producing at least one ethanol effluent, an acetaldehyde effluent and a water effluent;
F) a first butadiene purification step comprising at least one gas-liquid scrubbing section the bottom of which is supplied with the unrefined butadiene effluent obtained from B) and the head of which is supplied with a stream of water which may be a stream of water with an origin external to said butadiene production process and/or a fraction of the water effluent obtained from step E), said scrubbing section producing an overhead pre-purified butadiene effluent and a spent water effluent from the bottom;
G) a subsequent butadiene purification step supplied with at least said pre-purified butadiene effluent obtained from said step F), and producing at least one purified butadiene effluent.

2. The process according to claim 1, wherein the pre-purified butadiene effluent obtained from step F) undergoes a second butadiene purification step Fbis) before being supplied to the subsequent butadiene purification step G), said step Fbis) comprising at least one scrubbing section the bottom of which is supplied with said pre-purified butadiene effluent obtained from F), and the head of which is supplied with an absorbent solution.

3. The process according to claim 2, wherein said absorbent solution is an aqueous solution with a pH of more than 10, adjusted by adding sodium or potassium hydroxide.

4. The process according to claim 2, wherein said absorbent solution is an aqueous sodium or potassium bisulphite solution the pH of which is in the range 5 to 8.

5. The process according to claim 2, wherein said absorbent solution is an aqueous solution containing a compound from the hydrazine family.

6. The process according to one of claims 1 to 5, wherein step D) comprises at least one scrubbing/back-scrubbing section, a section for the distillation of light brown oils, and a section for the distillation of heavy brown oils, said scrubbing/back-scrubbing section being supplied at an intermediate point with said ethanol/acetaldehyde/water effluent obtained from step B), at the bottom with a hydrocarbon effluent and overhead with a fraction of the water effluent obtained from step E) and producing at least one hydrocarbon scrubbing extract and an ethanol/acetaldehyde/water raffinate, said light brown oil distillation being supplied with said hydrocarbon scrubbing extract and producing, as a distillate, a light brown oil effluent, and a hydrocarbon residue, said heavy oils distillation section being supplied with a fraction in the range 5% to 30% of the total flow rate of said hydrocarbon residue and producing a hydrocarbon distillate and, as a residue, a heavy brown oil effluent, said hydrocarbon distillate and the untreated fraction of said hydrocarbon residue being mixed in order to constitute the hydrocarbon effluent supplying said scrubbing/back-scrubbing section.

7. The process according to one of claims 1 to 6, wherein said reaction section of said step A) is further supplied with a fraction of said ethanol feed.

8. The process according to one of claims 1 to 7, wherein said step A) is further supplied with an external acetaldehyde stream.

9. The process according to one of claims 1 to 8, wherein the purge stream of said step A) is transformed in a dedicated hydrogenation step to produce ethanol which will then be sent to said reaction section of said step A).

10. The process according to one of claims 1 to 9, wherein the ethanol stream enriched in butadiene withdrawn from the bottom of said gas-liquid scrubbing section of step B) is mixed with the liquid effluent obtained from step A) such as to form the supply for said distillation section of step B).

11. The process according to one of claims 1 to 9, wherein the ethanol stream enriched in butadiene withdrawn from said gas-liquid scrubbing section of step B) constitutes the supply for said distillation section of step B), the liquid effluent obtained from step A) then being directly supplied to the step D) for elimination of liquid impurities and brown oils.

12. The process according to claim 6, wherein said scrubbing/back scrubbing section of said step D) is supplied with said ethanol/acetaldehyde/water effluent obtained from step B), at an intermediate point, as a mixture with the spent water effluent obtained from step E), the alcohol-containing effluent obtained from step C) and/or as a mixture with a fraction of the spent water effluent obtained from step F).

13. The process according to one of claims 1 to 12, wherein said section E) is further supplied with a fraction of the ethanol feed.

14. The process according to one of claims 1 to 13, wherein said step G) comprises at least one drying section, a cryogenic distillation section and a section for butadiene/butenes separation by liquid-liquid extraction.

15. The process according to one of claims 1 to 14, wherein said step G) comprises at least one distillation and an extractive distillation.
